Europäisches Patentamt

European Patent Office

Office européen des brevets

(19)

(11) Veröffentlichungsnummer: **0 310 541 B1**

## (12) EUROPÄISCHE PATENTSCHRIFT

(45) Veröffentlichungstag der Patentschrift: **09.12.92**

(21) Anmeldenummer: **88730221.4**

(22) Anmeldetag: **30.09.88**

(51) Int. Cl.5: **A61K 31/565**, //(A61K31/565, 31:135)

(54) Antigestagen- und antiöstrogenwirksame Verbindungen zur Geburtseinleitung und zum Schwangerschaftsabbruch sowie zur Behandlung gynäkologischer Störungen.

(30) Priorität: **01.10.87 DE 3733478**

(43) Veröffentlichungstag der Anmeldung:
**05.04.89 Patentblatt 89/14**

(45) Bekanntmachung des Hinweises auf die Patenterteilung:
**09.12.92 Patentblatt 92/50**

(84) Benannte Vertragsstaaten:
**AT BE CH DE ES FR GB GR IT LI LU NL SE**

(56) Entgegenhaltungen:
**EP-A- 0 139 608**
**EP-A- 0 219 447**
**EP-A- 0 266 303**
**DE-A- 3 323 321**
**US-A- 4 670 426**

**Biochem. and Biophys. Res. Comm. 1987,**
**Band 145, S. 706-11, Hissan et al.**

**Cancer Res. 1987, Band 47, S. 1441-8, Bardon**
**et al.**

(73) Patentinhaber: **SCHERING AKTIENGESELL-**
**SCHAFT Berlin und Bergkamen**
**Müllerstrasse 170/178 Postfach 65 03 11**
**W-1000 Berlin 65(DE)**

(72) Erfinder: **Elger, Walter, Dr.**
**Schorlemerallee 12B**
**W-1000 Berlin 33(DE)**
Erfinder: **Beier, Sybille, Dr.**
**Uhlandstrasse 121**
**W-1000 Berlin 31(DE)**
Erfinder: **Fähnrich, Marianne**
**Letteallee 48**
**W-1000 Berlin 51(DE)**
Erfinder: **Kosub, Beate**
**Forchbacher Strasse 51**
**W-1000 Berlin 37(DE)**
Erfinder: **Chwalisz, Krzysztof, Dr.**
**Luzerner Strasse 1d**
**W-1000 Berlin 45(DE)**
Erfinder: **Hasan, Syed Hamiduddin, Dr.**
**Angerburger Allee 37**
**W-1000 Berlin 19(DE)**

**Beschreibung**

Die vorliegende Erfindung betrifft die Verwendung mindestens einer Verbindung mit antigestagener (AG) und mindestens einer Verbindung mit antiöstrogener (AÖ) Wirkung zur Herstellung von Arzneimitteln zur Geburtseinleitung, zum Schwangerschaftsabbruch sowie zur Behandlung gynäkologischer Störungen.

Um Gefahren für Mutter und/oder Kind abzuwenden, ist es manchmal erforderlich, eine Geburt künstlich einzuleiten oder eine Schwangerschaft vorzeitig zu beenden. Dafür stehen chirurgische Techniken und pharmakologische Methoden zur Verfügung.

Eine mögliche pharmakologische Methode ist die vaginale oder intramuskuläre Applikation von Prostaglandinen, die im Falle des Schwangerschaftsabbruchs im 1. oder 2. Schwangerschaftrimester (Contraception 1983, Vol. 27, 51-60 und Int. J. Gynaecol. Obstet. 1982, Vol. 20, 383-386) vorgenommen wird.

Der Vorteil der Prostaglandin-Anwendung ist ihre Einsatzmöglichkeit über einen langen Zeitraum der Schwangerschaft. Als Nachteile der Prostaglandine sind akute Nebenwirkungen wie Schmerzen und Übelkeit zu nennen. Außerdem liegt die Erfolgsrate im Falle des Schwangerschaftsabbruchs in fortgeschrittenen Phasen der Schwangerschaft auch bei einer längeren Dauer der Prostaglandinbehandlung nicht über 90 %.

Eine andere Möglichkeit der Beendigung einer Schwangerschaft besteht in der Applikation eines Antigestagens (Med. et Hyg. 1982, Vol. 40, 2087-2093). Antigestagene sind besser verträglich als Prostaglandine, haben aber eine geringere Wirksamkeit, eine größere Latenz und individuelle Variabilität des Wirkungseintritts im Vergleich mit den Prostaglandinen. Außerdem wurde in der Klinik beobachtet, daß sie eine Tendenz zu z.T. schweren Blutungen aufweisen.

Die gemeinsame Verwendung von Prostaglandinen und Antigestagenen (EP 84730108.2) bringt zwar unbestreitbare Vorteile gegenüber der alleinigen Applikation der einzelnen Wirkstoffe (vor allem Reduktion der jeweiligen Wirkstoffmenge), löst aber z.B. nicht die Probleme, die generell bei der Verwendung von Prostaglandinen auftreten: Unerwünschte Nebenwirkungen wie gastrointestinale Effekte oder Uterus-Schmerzen, die Behandlung muß stationär erfolgen, Lagerung und Haltbarkeit des Arzneimittels sind aufgrund mangelnder Stabilität begrenzt und/oder aufwendig, die anwenderfreundlichste Applikationsform, nämlich die orale, ist nicht möglich und damit auch nicht die Kombination beider Wirkstoffe in einer Tablette, Pille oder einem Dragee.

Pharmazeutische Zusammensetzungen zur post-coitalen Fertilitätskontrolle, die einen kompetitiven Progesteronantagonisten (Antigestagen) sowie einen Progesteron- und Östrogensyntheseblocker enthalten, sind bereits im US-Patent 4,670,426 beschrieben. Als typische Vertreter für den zu verwendenden kompetitiven Progesteronantagonisten sind Fuocinolonacetonid, Triamcinolonacetonid, Steroide mit einem zyklischen 16, 17-Acetal mit Aceton und 17$\beta$-Hydroxy-11$\beta$ -(4-dimethylaminophenyl-1)-17$\alpha$-prop-1-inyl)-estra-4,9-dien-3-on und äquivalente Derivate erwähnt. Der typische Gehalt liegt dabei zwischen 20 und 50 mg. Als Beispiele für den Progesteron- und Östrogensyntheseblocker sind Aminoglutethimid, 2$\alpha$-Cyano-4,4,17$\alpha$-trimethyl-5-androst-5-en-17$\beta$-ol-3-on, 20, 25-Diazocholesterol und Verbindungen mit äquivalenter Aktivität angeführt, und zwar in einer Dosis von 300 bis 1000 mg. Die Anwendung der Zusammensetzung hat gemäß US-Patent 4, 670,426 möglichst früh innerhalb der ersten Woche nach dem Geschlechtsverkehr über einen Zeitraum von 3 Tagen zu erfolgen; am besten sollte die Behandlung 2 bis 6 Tage fortgesetzt werden. Die Verhinderung der Nidation und somit einer Schwangerschaft wird durch den synergistischen Effekt bei der gemeinsamen Anwendung der beiden Bestandteile der Zusammensetzung bewirkt, und zwar mit einer Erfolgsrate in der Größenordnung von 90 % oder mehr.

Für die Geburtseinleitung, den Schwangerschaftsabbruch und die Behandlung gynäkologischer Störungen sind antiöstrogenwirksame Verbindungen bisher nicht vorgeschlagen worden.

Der Erfindung liegt die Aufgabe zugrunde, Arzneimittel für die angegebenen Verwendungen bereitzustellen, die die oben genannten Nachteile nicht aufweisen, gleichzeitig eine hohe, möglichst höhere Wirksamkeit im Vergleich zu den bekannten Mitteln haben und weniger Nebenwirkungen als diese aufweisen.

Diese Aufgabe wird durch die Erfindung gelöst.

Es wurde gefunden, daß bei der Verwendung von AG und AÖ die Wirksamkeit der Einzelkomponenten dramatisch verstärkt wird, wobei für die Beendigung der Schwangerschaft die AÖ-Komponente allein nicht wirksam ist.

Die erfindungsgemäß hergestellten Arzneimittel sind dabei nicht nur in hohem Maße zur Geburtseinleitung und zum Schwangerschaftsabbruch geeignet; sie finden zusätzliche Verwendung als Mittel gegen Endometriose und Dysmenorrhoe. Es konnte erstmalig gezeigt werden, daß durch die erfindungsgemäß hergestellten Arzneimittel die Wirkungen von AG und AÖ sich gegenseitig verstärken.

Das Gewichtsverhältnis beider Komponenten kann dabei in weiten Grenzen variiert werden. So können sowohl gleiche Mengen AG und AÖ als auch ein Überschuß eines der beiden Komponente eingesetzt werden. AG und AÖ werden gemeinsam, getrennt, gleichzeitig und/oder zeitlich abgestuft (sequential), in einem Gewichtsverhältnis von im wesentlichen 1:50 bis 50:1, vorzugsweise 1:25 bis 25:1, und insbesondere 1:10 bis 10:1 verwendet. Die gleichzeitige Gabe ist bevorzugt. Im Falle der sequentiellen Gabe kann die als zweite gegebene Verbindung zu jeder Zeit nach Gabe der zuerst applizierten Verbindung gegeben werden, solange sie noch in der Patientin gleichzeitig mit einer wirksamen Menge der zuerst applizierten Verbindung bioverfügbar wird. Beispielsweise kann AÖ ab dem 2. Tag nach der Applikation von AG gegeben werden, wobei am 3. und 4. Tag dann außerdem noch sowohl AG als auch AÖ appliziert werden können.

Die gemeinsame Behandlung mit AG und AÖ für die Beendigung der Schwangerschaft erfolgt in der Regel über 1 bis 4, vorzugsweise 1 bis 2 Tage. Gemäß der vorliegenden Erfindung wird zum Abbruch der Gravidität und zur Einleitung der Geburt die Kombination AG und AÖ in jedem Fall nach der Nidation appliziert, vorzugsweise im zweiten oder dritten Drittel der Gravidität, im Fall der Geburtseinleitung kurz bevor oder am Geburtstermin.

Vorzugsweise können AG und AÖ kombiniert in einer Dosiseinheit appliziert werden.

Als Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage :
11$\beta$-[(4-N,N-Dimethylamino)-phenyl]-17$\beta$-hydroxy-17$\alpha$-propinyl-4,9(10)-estradien-3-on (RU-38486, EP-A 0057 115),
11B-[(4-N,N-Dimethylamino)-phenyl]-17$\beta$-hydroxy-18-methyl-17$\alpha$-propinyl-4,9(10)-estradien-3-on und
11$\beta$-[(4-N,N-Dimethylamino)-phenyl]-17a$\beta$-hydroxy-17a$\alpha$-propinyl-D-homo-4,9(10), 16-estratrien-3-on (EP-A 0057 115) ;
ferner
11$\beta$-p-Methoxyphenyl-17$\beta$-hydroxy-17$\alpha$-ethinyl-4,9(10)-estradien-3-on (Steroids 37 (1981) 361-382) und
11$\beta$-(4-Dimethylaminophenyl)-17$\alpha$-hydroxy-17$\beta$-(3-hydroxypropyl)-13$\alpha$-methyl-4,9-gonadien-3-on (EP-A 0129 499).

Die Antigestagene werden gemäß vorliegender Erfindung in Mengen eingesetzt, die unterhalb der sonst für den Schwangerschaftsabbruch üblichen (und für eine 100 %ige Erfolgsrate trotzdem oft nicht ausreichenden) Mengen liegen. Im allgemeinen wird man mit 10 - 200 mg 11$\beta$-[4-N,N-Dimethylamino)-phenyl]-17$\alpha$-hydroxy-17$\beta$-(3-hydroxypropyl)-13$\alpha$-methyl-4,9(10)-gonadien-3-on pro Tag oder einer biologisch äquivalenten Menge eines anderen Antigestagens auskommen.

Die Dosierung bei der Behandlung von gynäkologischen Störungen liegt bei 1 - 1000 mg 11$\beta$-[4-N,N-Dimethylamino)-phenyl-17$\alpha$-hydroxy-17$\beta$-(3-hydroxypropyl)-13$\alpha$-methyl-4,9(10)-gonadien-3-on pro Tag oder - einer biologisch äquivalenten Menge eines anderen Antigestagens.

Als antiöstrogen wirkende Verbindungen kommen Antiöstrogene und Aromatasehemmer infrage. Antiöstrogene und Aromatasehemmer gemäß vorliegender Erfindung können sowohl von Steroiden abgeleitet oder nicht-steroidale Verbindungen sein. Unter antiöstrogen wirkenden Verbindungen gemäß vorliegender Erfindung sollen nur solche Verbindungen verstanden werden, die möglichst selektiv wirken, d.h. die im wesentlichen nur die Wirkung von Östrogenen hemmen und/oder deren Konzentration senken.
Die Antiöstrogene wirken als kompetitive Östrogenantagonisten, indem sie Östrogen vom Rezeptor verdrängen, während Aromatasehemmer die Biosynthese des Östrogens unterdrücken. Verbindungen vom Typ des Aminoglutethimids, d. h. in der 3-Stellung alkylierte 3-(4-Aminophenyl)-piperidin-2,6-dione und andere, die außer auf den Östrogenspiegel auch auf andere Sexualhormonserumkonzentrationen erniedrigend wirken, sind gemäß vorliegender Erfindung als Antiöstrogen wirksame Verbindungen nicht geeignet.

Als Antiöstrogene kommen alle gebräuchlichen Antiöstrogene in Betracht. Sie können etwa in gleichen Mengen eingesetzt werden wie die bereits im Handel befindlichen Antiöstrogene, das heißt die tägliche Dosis beträgt etwa 5 - 100 mg für Tamoxifen oder biologisch äquivalente Mengen eines anderen Antiöstrogens. Als nicht-steroidale Antiöstrogene seien beispielsweise genannt:

Tamoxifen = (Z)-2-[p-(1,2-Diphenyl-1-butenyl)-phenoxy]-N,N-dimethyläthylamin,
Nafoxidin = 1 -2-[4-(6-Methoxy-2-phenyl-3,4-dihydro-1-naphthyl)-phenoxy]-äthyl -pyrrolidin, hydrochlorid,
Mer 25 = 1-[p(2-Diäthylaminoäthoxy)-phenyl]-2-(p-methoxyphenyl)-1-phenyläthanol und
Verbindungen vom 1,1,2-Triphenylbut-1-en-Typ, insbesondere das 1,1-Bis-(3´-acetoxyphenyl)-2-phenyl-but-1-en [J. Cancer Res. Clin. Oncol., (1986), 112, S. 119-124].

Ferner kommen als steroidale Antiöstrogene infrage:
11$\alpha$-Methoxy-17$\alpha$-äthinyl-1,3,5(10)-östratrien-3,17$\beta$-diol und 16$\beta$-Äthylestradiol.

Als Aromatasehemmer sind alle Verbindungen geeignet, die als Substrat für die Aromatase infrage kommen, wie beispielsweise das in der deutschen Offenlegungsschrift 33 22 285 beschriebene
1-Methyl-androsta-1,4-dien-3,17-dion,
das in Journal of Clinical Endocrinology and Metabolism. 49, 672 (1979) beschriebene
Testolacton (17a-Oxa-D-homoandrost-1,4-dien-3,17-dion),
die in "Endocrinology" 1973, Vol. 92, No. 3, Seite 874 beschriebenen Verbindungen
Androsta-4,6-dien-3,17-dion,
Androsta-4,6-dien-17$\beta$-ol-3-on-acetat,
Androsta-1,4,6-trien-3,17-dion,
4-Androsten-19-chlor-3,17-dion,
4-Androsten-3,6,17-trion,
die in der deutschen Offenlegungsschrift 31 24 780 beschriebenen
19-alkynylierten Steroide,
die in der deutschen Offenlegungsschrift 31 24 719 beschriebenen
10-(1,2-Propadienyl)-steroide,
die in der europäischen Patentanmeldung, Veröffentlichungsnummer 100 566 beschriebenen
19-Thio-androstanderivate,
das in "Endocrinology" 1977, Vol. 100, No. 6, Seite 1684 und der US-Patentschrift 4,235,893 beschriebene
4-Androsten-4-ol-3,17-dion und dessen Ester,
die in der deutschen Offenlegungsschrift 35 39 244 beschriebenen
1-Methyl-15$\alpha$-alkyl-androsta-1,4-dien-3,17-dione,
die in der deutschen Offenlegungsschrift 36 44 358 beschriebenen
10$\beta$-Alkinyl-4,9(11)-östradien-derivate und das in der europäischen Patentanmeldung 0250262 beschriebene
1,2$\beta$-Methylen-6-methylen-4-androsten-3,17-dion.

Als nicht-steroidaler Aromatasehemmer sei beispielsweise das [4-(5,6,7,8-Tetrahydroimidazo[1,5$\alpha$]-pyridin-5-yl)benzonitril-monohydrochlorid] erwähnt (Cancer Res., 48, S. 834-838, 1988).

Im allgemeinen werden täglich 10 - 200 mg 1-Methyl-androsta-1,4-dien-3,17-dion bzw. biologisch äquivalente Dosen von anderen Aromatasehemmern für die Einleitung der Geburt oder den Abbruch der Schwangerschaft eingesetzt.

Die Dosierung bei der Behandlung von gynäkologischen Störungen liegt bei 1 - 1000 mg 1-Methyl-androsta-1,4-dien-3,17-dion pro Tag oder biologisch äquivalenten Dosen von anderen Aromatasehemmern.

Antigestagen- und antiöstrogenwirksame Verbindungen können zum Beispiel lokal, topisch, enteral oder parenteral appliziert werden.

Für die bevorzugte enterale Applikation kommen insbesondere Tabletten, Dragees, Kapseln, Pillen, Suspensionen oder Lösungen infrage, die in üblicher Weise mit den in der Galenik üblichen Zusätzen und Trägersubstanzen hergestellt werden können. Für die lokale oder topische Anwendung kommen beispielsweise Vaginalzäpfchen oder transdermale Systeme wie Hautpflaster infrage.

Eine AG-Dosiseinheit enthält etwa 1 - 200 mg 11$\beta$-[(4-N,N-Dimethylamino)-phenyl]-17$\alpha$-hydroxy-17$\beta$-(3-hydroxypropyl)-13$\alpha$-methyl-4,9(10)-gonadien-3-on oder eine biologisch äquivalente Menge eines anderen Antigestagens.

Eine AÖ-Dosiseinheit enthält 1 - 100 mg Tamoxifen oder 10 - 200 mg 1-Methyl-androsta-1,4-dien-3,17-dion oder eine biologisch äquivalente Menge einer anderen antiöstrogen wirksamen Verbindung.

**Beispiel 1**

| 10,0 mg | 11$\beta$-[(4-N,N-Dimethylamino)-phenyl]-17$\alpha$-hydroxy-17$\beta$-(3-hydroxypropyl)-13$\alpha$-methyl-4,9-gonadien-3-on |
| 140,5 mg | Laktose |
| 69,5 mg | Maisstärke |
| 2,5 mg | Polyvinylpyrrolidon 25 |
| 2,0 mg | Aerosil |
| 0,5 mg | Magnesiumstearat |
| 225,0 mg | Gesamtgewicht der Tablette |

**Beispiel 2**

| | |
|---|---|
| 50,0 mg | 1-Methyl-androsta-1,4-dien-3,17-dion |
| 115,0 mg | Laktose |
| 50,0 mg | Maisstärke |
| 2,5 mg | Poly-N-Vinylpyrrolidon 25 |
| 2,0 mg | Aerosil |
| 0,5 mg | Magnesiumstearat |
| 220,0 mg | Gesamtgewicht der Tablette |

**Beispiel 3**

| | |
|---|---|
| 25,0 mg | 1-Methyl-androsta-1,4-dien-3,17-dion |
| 25,0 mg | $11\beta$-[(4-N,N-Dimethylamino)-phenyl]-$17\alpha$-hydroxy-$17\beta$-(3-hydroxypropyl)-$13\alpha$-methyl-4,9--gonadien-3-on |
| 115,0 mg | Laktose |
| 50,0 mg | Maisstärke |
| 2,5 mg | Poly-N-Vinylpyrrolidon 25 |
| 2,0 mg | Aerosil |
| 0,5 mg | Magnesiumstearat |
| 220,0 mg | Gesamtgewicht der Tablette, die in üblicher Weise auf einer Tablettenpresse hergestellt wird. Gegebenenfalls können auch die erfindungsgemäßen Wirkstoffe mit jeweils der Hälfte der oben angegebenen Zusätze getrennt zu einer Zweischichtentablette gepreßt werden. |

**Beispiel 4**

| | |
|---|---|
| 10,0 mg | Tamoxifen |
| 10,0 mg | $11\beta$-[(4-N,N-Dimethylamino)-phenyl]-$17\alpha$-hydroxy-$17\beta$-(3-hydroxypropyl)-$13\alpha$-methyl-4,9-g-onadien-3-on |
| 135,0 mg | Laktose |
| 60,0 mg | Maisstärke |
| 2,5 mg | Poly-N-Vinylpyrrolidon 25 |
| 2,0 mg | Aerosil |
| 0,5 mg | Magnesiumstearat |
| 220,0 mg | Gesamtgewicht der Tablette, die in üblicher Weise auf einer Tablettenpresse hergestellt wird. Gegebenenfalls können auch die erfindungsgemäßen Wirkstoffe mit jeweils der Hälfte der oben angegebenen Zusätze getrennt zu einer Zweischichtentablette gepreßt werden. |

**Beispiel 5**

**Zusammensetzung einer öligen Lösung:**

| 100,0 mg | Tamoxifen |
|---|---|
| 343,4 mg | Rizinusöl |
| 608,6 mg | Benzylbenzoat |
| 1052,0 mg | = 1 ml |
| Die Lösung wird in eine Ampulle gefüllt. | |

**Beispiel 6**

**Zusammensetzung einer öligen Lösung:**

| 55,0 mg | 1-Methyl-androsta-1,4-dien-3,17-dion |
|---|---|
| 55,0 mg | 11$\beta$-[(4-N,N-Dimethylamino)-phenyl]-17$\alpha$-hydroxy-17$\beta$-(3-hydroxyp-ropyl)-13$\alpha$-methyl-4,9-gonadien-3-on |
| 343,4 mg | Rizinusöl |
| 608,6 mg | Benzylbenzoat |
| 1062,0 mg | = 1 ml |
| Die Lösung wird in eine Ampulle gefüllt. Die erfindungsgemäßen Wirkstoffe können auch mit jeweils der Hälfte der oben angegebenen Zusätze getrennt in zwei Kammern abgefüllt werden. | |

**Pharmakologische Beobachtungen**

Für die Versuche an graviden Meerschweinchen wurden die antiöstrogen-wirksamen Verbindungen Tamoxifen und 1-Methyl-1,4-androstadien-3,17-dion sowie die antigestagen-wirksame Verbindung 11$\beta$-[(4-N,N-dimethylamino)-phenyl]-17$\alpha$-hydroxy-17$\beta$-(3-hydroxypropyl)-13$\alpha$-methyl-4,9(10)-gonadien-3-on als Modellsubstanzen ausgewählt. Die geprüften Dosierungen sind der Abbildung zu entnehmen.

**Untersuchungen an graviden Meerschweinchen**

Prüfung der Kombination
-Versuchsbeschreibung -

Gravide Meerschweinchen mit einem Körpergewicht von ca. 800 g wurden am 42. Tag der Schwangerschaft in den Versuch genommen (der 2. Tag der Vaginalöffnung in der Anpaarungsphase wurde als erster Tag der Gravidität gerechnet). Vor Versuchsbeginn wurde die Gravidität durch Palpation kontrolliert. Die Behandlung mit den ausgewählten Prüfsubstanzen bzw. der Kombination erfolgte durch tägliche subcutane Applikation am 43. und 44. Tag der Gravidität. Die Verbindungen wurden dazu in Benzylbenzoat + Rizinusöl (Mischungsverhältnis 2 + 4,5) gelöst und die tägliche Dosis in einem Volumen von 1,0 ml s.c. injiziert. Das eventuelle Ausstoßen von Früchten wurde unter und nach der Behandlung mehrfach täglich kontrolliert. Am 50. Tag der Gravidität wurden die Tiere getötet. Die Uteri wurden inspiziert und die Foeten befundet.

Ergebnisse

Die Ergebnisse der Versuche zur Abortinduktion beim graviden Meerschweinchen bei kombinierter Verabreichung von antigestagen- und antiöstrogen-wirksamen Verbindungen sind der Abbildung zu entnehmen.

**Antiöstrogenwirksame Verbindungen (AÖ)**

Mit einer Dosis von 10 mg/Tag s.c. war 1-Methyl-1,4-androstadien-3,17-dion total inaktiv im Hinblick auf abortive Wirkukng (s. Abbildung).

**Antigestagene (AG)**

Mit dem Antigestagen A war eine Unterbrechung einer bestehenden Schwangerschaft mit 10 mg/Tag s.c. bei ca. 50 % der behandelten Tiere zu erzielen. Die Aborte erfolgten mit einer bis zu 4tägigen Latenz vom Behandlungsbeginn (s. Abbildung).

**AG/AÖ-Kombination**

Die Kombinationen von nur zu 50 % wirksamen Antigestagendosen (10,0 mg A/Tag s.c.) mit einer unwirksamen Tamoxifen- oder 1-Methyl-1,4-androstadien-3,17-dion-Dosis von 10 mg/Tag s.c. führten zu einer 100 %igen Abortrate und zu einem schnelleren Auftreten der Aborte. Die Latenzzeit ist im Falle des 1-Methyl-1,4-androstadien-3,17-dions auf 0,5 Tage verkürzt worden.

**Vergleichende Betrachtung der abortiven Wirkung von antigestagen- und antiöstrogenwirksamen Verbindungen beim graviden Meerschweinchen**

Kumulative

Abortrate

A          10mg/d s.c. d43+44
□—□ + Tamoxifen  10mg/d s.c. d43+44
           n = 4

A          10mg/d s.c. d43+44
○—○ + B    10mg/d s.c. d43+44
           n = 4

□- -□  A   10mg/d s.c. d43+44
           n = 9

△—△  B     10mg/d s.c. d43+44
           n = 5

A = 11ß-[(4-N,N-Dimethylamino)-phenyl]-17α-hydroxy-17ß-(3-hydroxypropyl)-13α-methyl-4,9(10)-gonadien-3-on

B = 1-Methyl-1,4-androstadien-3,17-dion

n = Zahl der Tiere

**Patentansprüche**

1. Verwendung mindestens einer Verbindung mit antigestagener (AG) und mindestens einer Verbindung mit antiöstrogener (AÖ) Wirkung zur Herstellung von Arzneimitteln zur Geburtseinleitung, zum Schwangerschaftsabbruch sowie zur Behandlung gynäkologischer Störungen.

2. Verwendung von AG und AÖ nach Anspruch 1 in einem Gewichtsverhältnis von 1:50 bis 5:1.

3. Verwendung von AG und AÖ nach Anspruch 1 in getrennten Dosiseinheiten.

4. Verwendung von AG und AÖ nach Anspruch 1 in einer gemeinsamen Dosiseinheit.

5. Verwendung von AG und AÖ nach Anspruch 1, dadurch gekennzeichnet, daß als AG 1 bis 200 mg 11ß-[(4-N,N-Dimethylamino)-phenyl]-17$\alpha$-hydroxy-17ß-(3-hydroxypropyl)-13$\alpha$-methyl-4,9(10)-gonadien-3-on oder eine biologisch äquivalente Menge eines anderen AG in einer Dosiseinheit verwendet wird.

6. Verwendung von AG und AÖ nach Anspruch 1, dadurch gekennzeichnet, daß als AÖ 1 bis 100 mg Tamoxifen oder eine biologisch äquivalente Menge eines anderen AÖ in einer Dosiseinheit verwendet wird.

7. Verwendung von AG und AÖ nach Anspruch 1, dadurch gekennzeichnet, daß als AÖ 10 bis 200 mg 1-Methyl-androsta-1,4-dien-3,17-dion oder eine biologisch äquivalente Menge eines anderen AÖ in einer Dosiseinheit verwendet wird.

## Claims

1. Use of at least one compound having antigestagen activity (AG) and at least one compound having antioestrogen activity (AO) for the preparation of medicaments for inducing labour, for terminating pregnancy and for the treatment of gynaecological disorders.

2. Use of AG and AO according to claim 1 in a ratio by weight of 1:50 to 5:1.

3. Use of AG and AO according to claim 1 in separate dosage units.

4. Use of AG and AO according to claim 1 together in a single dosage unit.

5. Use of AG and AO according to claim 1, characterised in that the AG used in a dosage unit is from 1 to 200 mg of 11ß-[(4-N,N-dimethylamino)-phenyl]-17$\alpha$-hydroxy-17ß-(3-hydroxypropyl)-13$\alpha$-methyl-4,9(10)-gonadien-3-one or a biologically equivalent amount of a different AG.

6. Use of AG and AO according to claim 1, characterised in that the AO used in a dosage unit is from 1 to 100 mg of tamoxifen or a biologically equivalent amount of a different AO.

7. Use of AG and AO according to claim 1, characterised in that the AO used in a dosage unit is from 10 to 200 mg of 1-methyl-androsta-1,4-dien-3,17-dione or a biologically equivalent amount of a different AO.

## Revendications

1. Application d'au moins un composé ayant une action anti-progestative (AP) et d'au moins un composé ayant une action anti-estrogène (AE) pour la fabrication de médicaments destinés à induire une naissance, à interrompre une grossesse ainsi qu'à traiter des troubles gynécologiques.

2. Application d'un AP et d'un AE selon la revendication 1, dans un rapport pondéral compris entre 1:50 et 5:1.

3. Application d'un AP et d'un AE selon la revendication 1, dans des unités de prise séparées.

4. Application d'un AP et d'un AE selon la revendication 1, dans une unité de prise commune.

5. Application d'un AP et d'un AE selon la revendication 1, caractérisée en ce qu'on utilise, comme AP, de 1 à 200 mg de 11ß-[4-(N,N-diméthylamino)-phényl]-17$\alpha$-hydroxy-17ß-(3-hydroxypropyl)-13$\alpha$-méthyl-

4,9(10)-gonadiène-3-one ou une quantité biologiquement équivalente d'un autre AP, dans une unité de prise.

6. Application d'un AP et d'un AE selon la revendication 1, caractérisée en ce qu'on utilise, comme AE, de 1 à 100 mg de tamoxifène, ou une quantité biologiquement équivalente d'un autre AE, dans une unité de prise.

7. Application d'un AP et d'un AE selon la revendication 1, caractérisée en ce qu'on utilise, comme AE, de 10 à 200 mg de 1-méthyl-androsta-1,4-diène-3,17-dione, ou une quantité biologiquement équivalente d'un outre AE, dans une unité de prise.